# EUROPEAN PATENT APPLICATION

(11) **EP 1 586 313 A1**
(43) Date of publication of application: **19.10.2005**
(21) Application number: 04008388.3
(22) Date of filing: 07.04.2004
(51) Int. Cl.: A61K 31/165, A61K 9/26, A61K 9/28

(54) **Pharmaceutical composition with metoclopramide and process for its preparation**

(71) Applicant: The Jordanian Pharmaceutical Manufacturing Co. Ltd., 11710 Naor (JO)
(72) Inventor: Adnan, Badwan, Dr., Amman 11185 (JO); Enass, Hassan, I, Amman 11814 (JO); Basam, Amr, Amman 11181 (JO)
(74) Representative: Winkler, Andreas Fritz Ernst

(57) **Abstract**

The present invention relates to a pharmaceutical composition comprising at least one hydrophilic polymer as a matrix and metoclopramide dispersed therein; as well as to a process for its preparation.

## Description

The present invention relates to a pharmaceutical composition with metoclopramide for oral administration and a process for its preparation.

Metoclopramide hydrochloride {4-amino-5-chloro-N-(2-diethylaminoethyl)-2-methoxybenzamide hydrochloride monohydrate} is commonly used in a variety of gastrointestinal (GI) disorders, but principally for the management of GI motility disorders, especially gastric stasis, for the management of gastroesophageal reflux and for the prevention of cancer chemotherapy-induced nausea and vomiting. The drug is highly water-soluble and is rapidly absorbed after oral administration. It has a short biological half-life and is usually administered in a dose of 10-15 mg given up to four times daily in order to maintain effective concentration throughout the day.

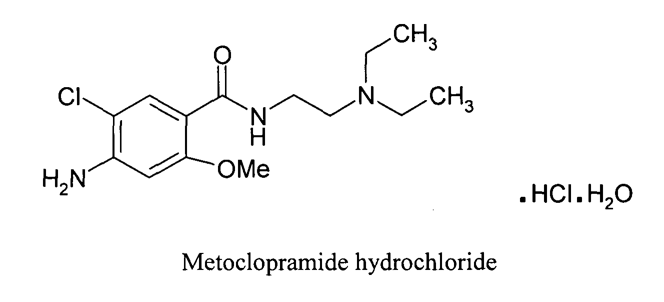

Extrapyramidal effects may occur at therapeutic doses in any age group. However, they occur more frequently in children and young adults, and at the higher doses used in prophylaxis of vomiting due to cancer chemotherapy. Extrapyramidal symptoms will surface as well if plasma level markedly exceed therapeutic levels (Ganza-Gonzalez, A., Anguiano-Igea, S., Otero-Espinar, F.J. and Blanco Mendez, J., (1999). Chitosan and chondroitin microspheres for oral administration controlled release of metoclopramide. *European Journal of Pharmaceutics and Biopharmaceutics,* 48:149-155).

It is therefore an object of the present invention to overcome the problems of the prior art and to provide a pharmaceutical composition with metoclopramide preventing high plasma concentrations and reducing the risk of suffering from extrapyramidal symptoms usually experienced with conventional dosage form treatments.

It is a further object to provide a process for the preparation of that pharmaceutical composition.

The first object is achieved by a pharmaceutical composition comprising at least one hydrophilic polymer as a matrix and metoclopramid dispersed therein.

Preferably, the polymer is a natural polysaccharide.

Most preferably, the polymer is chitosan, sodium alginate or a mixture thereof.

In one embodiment, metoclopramide is present in an amount of about 1 to about 50 weight percent, preferably about 5 to about 20 weight percent, based on the total weight of the pharmaceutical composition.

Further, the polymer may be present in an amount of about 10 to about 75 weight percent, preferably about 25 to about 75 weight percent, more preferably about 40 to about 60 weight percent, based on the total weight of the pharmaceutical composition.

Preferably, at least one processing aid is added.

Still preferred, the processing aid comprises filler, binder, glidant, lubricant and/or disintegrant.

In one embodiment the pharmaceutical composition comprises about 5 to about 30 weight percent, preferably about 15 to about 30 weight percent filler, about 1 to about 15 weight percent, preferably about 5 to about 15 weight percent binder, about 0.5 to about 3 weight percent, preferably 0.5 to about 2 weight percent glidant, about 0.5 to about 3 weight percent, preferably 0.5 to about 2 weight percent lubricant and about 5 to about 20 weight percent disintegrant.

Preferably, the composition is a capsule or a tablet.

More preferably, the tablet is film coated.

The second object is achieved by a process for preparing a pharmaceutical composition according to the invention, comprising the steps of mixing the polymer, metoclopramide and optionally processing aid(s), wet granulating the obtained mixture and compressing the obtained mixed granules into tablets.

It was surprising by found that when metoclopramide was placed in a matrix consisting of hydrophilic polymers the maximum plasma concentrations were found to be significantly lower than for conventional dosage forms.

This invention utilizes preferably natural polysaccharides such as chitosan and sodium alginate either as a combination or individually to be used as hydrophilic polymers to modify the release of metoclopramide HCl. The term "metoclopramide" used in the present application refers to all possible metoclopramide derivatives, such as metoclopramide hydrochloride monohydrate, which are suitable for oral administration and for treatment of GI disorders.

This invention presents a modified release formulation that provides twice daily administration, the adverse effects of metoclopramide on the central nervous system caused by high plasma peaks are thus avoided.

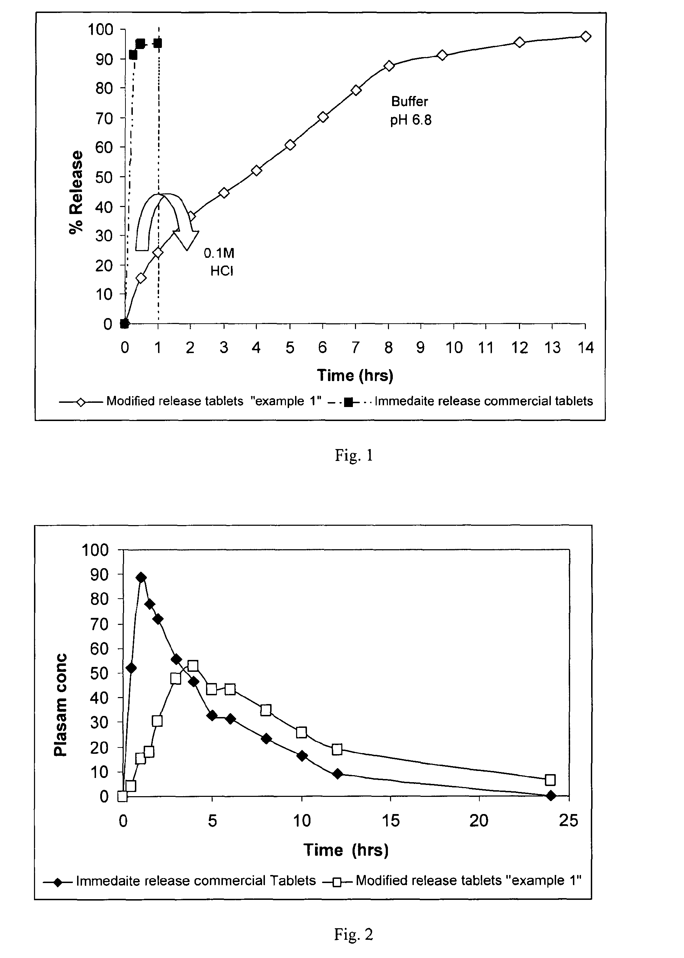

Additional advantages and features of the present invention are now illustrated in detail with reference to the accompanying drawing, wherein figure 1 illustrates the dependency on time for the release of metoclopramide of a conventional tablet and a tablet according to the invention, and
figure 2 illustrates a graph showing the dependency on time for the plasma concentration comparing a commercially available tablet and a tablet according to the present invention.

The pharmaceutical composition of the invention has been tested by in vivo and in vitro methods to test its effectiveness as a modified release formulation and safety in overcoming the side effects encountered with the conventional dosage forms.

The preparation of the inventive pharmaceutical composition may involve the wet granulation of metoclopramide in the presence of the release modifying hydrophilic polymer(s) preferably in the presence of processing aids. The processing aids may include a filler, a disintegrant, a binder, a glidant and a lubricant. The preparation may involve the sieving and appropriate mixing of metoclopramide, the release controlling hydrophilic polymer(s), filler(s), and the disintegrant. The powder mix is then granulated with the binder solution, until a granulation endpoint was reached. The wet mass was forced through a sieve, and then dried at 60°C for 3 hours. The dried granules are crushed, and the glidant is sieved and mixed with powder with 5 minutes. The lubricant is added and mixed for 1 minute. The mixed granules were then compressed on a single punch-tableting machine. The tablet may be film coated using an aqueous coating system. The following examples have been prepared according to the above procedure.

### Example 1

| **No.** | **Component** | **Function** | **Weight %** |
|---|---|---|---|
| 1 | Metoclopramide HCl | Active ingredient | 5-20 |
| 2 | Chitosan | Release controlling polymer | 20-30 |
| 3 | Sodium alginate | Release controlling polymer | 20-30 |
| 4 | Calcium hydrogen phosphate | Filler | 20-30 |
| 5 | Microcrystalline cellulose | Disintegrant | 5-20 |
| 6 | Povidone | Binder | 5-15 |
| 7 | Colloidal anhydrous silica | Glidant | 0.5-2.0 |
| 8 | Magnesium stearate | Lubricant | 0.5-2.0 |

### Example 2

| **No.** | **Component** | **Function** | **Weight %** |
|---|---|---|---|
| 1 | Metoclopramide HCl | Active ingredient | 5-20 |
| 2 | Chitosan | Release controlling polymer | 25-75 |
| 3 | Calcium hydrogen phosphate | Filler | 15-30 |
| 4 | Microcrystalline cellulose | Disintegrant | 5-20 |
| 5 | Povidone | Binder | 5-15 |
| 6 | Colloidal anhydrous silica | Glidant | 0.5-2.0 |
| 7 | Magnesium stearate | Lubricant | 0.5-2.0 |

### Example 3

| **No.** | **Component** | **Function** | **Weight %** |
|---|---|---|---|
| 1 | Metoclopramide HCl | Active ingredient | 5-20 |
| 2 | Sodium alginate | Release controlling polymer | 25-75 |
| 3 | Calcium hydrogen phosphate | Filler | 15-30 |
| 4 | Microcrystalline cellulose | Disintegrant | 5-20 |
| 5 | Povidone | Binder | 5-15 |
| 6 | Colloidal anhydrous silica | Glidant | 0.5-2.0 |
| 7 | Magnesium stearate | Lubricant | 0.5-2.0 |

The dissolution profile of the controlled release pharmaceutical composition (example 1) was tested vs. a conventional immediate release formulation present in the market. The conditions for the dissolution test are as follows: USP dissolution apparatus II (paddle), speed was kept at 50 rpm ± 2 rpm. The vessels were placed in a water bath regulated to maintain temperature of 37°C ± 0.5°C during the test. A fitted cover was used on the vessel to prevent any evaporation during the test time. Dissolution testing was preformed by testing the tablets for 1 hour in 600 ml of 0.1 M hydrochloric acid. The acidic media was then decanted and replaced with 600 ml of phosphate buffer pH 6.8. The dosage unit is allowed to sink to the bottom of the vessel before starting pedal movement. At specified time intervals, 4 ml aliquots were withdrawn, filtered through 0.45µm cellulose acetate filters, into vials and kept closed until analysis. At each time interval an aliquot equal in volume to the withdrawn sample was replaced, to maintain the original volume. The samples were analyzed spectrophotometrically by using the first derivative absorbance at 321.5 nm using the respective dissolution media as a blank. The results are shown in figure 1.

As can be seen from figure 1, the immediate release tablet show almost full release after 30 minutes meanwhile the tablet according to the present invention shows modified release over almost 12 hours. To further assess the effectiveness of this presented art, it was tested in vivo on 6 subjects, and it gave the following pharmacokinetics results:

**Table 1**

| Mean pharmacokinetic parameters of Metoclopramide following oral administration of the modified release and the immediate release tablets to six male volunteers | | |
|---|---|---|
| Parameter | Inventive release matrix "30 mg" | Immediate release tablets "10 mg" |
| Cₘₐₓ (ng/mL) | 63.36 ± 31.70 | 31.97 ± 10.90 |
| tₘₐₓ (hrs) | 4.50 ± 1.22 | 1.20 ± 0.45 |
| AUC_{0→t} (ng.hr/mL) | 609.30 ± 187.50 | 157.98 ± 58.3 |
| AUC_{0→∞} (ng.hr/mL) | 647.68 ± 205.60 | 172.10 ± 70.1 |
| t_{1/2} (hrs) | 6.43 ± 1.50 | 2.99 ± 0.8 |
| AUMC (ng.hr ²/mL) | 7874.34 ± 2050.20 | 948.98 ± 371.0 |
| MRT (hr) | 11.78 ± 0.90 | 5.54 ± 0.1 |
| Cₘₐₓ/AUC_{0→∞} (hr⁻¹) | 0.093 ± 0.03 | 0.186 ± 0.02 |
| Concentration at 8 h/Cₘₐₓ (%) | 55.3 ± 14.6 | 24.4 ± 5.7 |
| Concentration at 12 h/Cₘₐₓ (%) | 30.1 ± 5.6 | 9.30 ± 5.3 |
| **Note:** Data presented as mean ± S.D. | | |

The absorption of metoclopramide from the immediate release tablets of the prior art was rapid, the mean tₘₐₓ (time after which maximum concentration is achieved) was 1.20 h, whereas in the modified release tablets of the invention the mean tₘₐₓ was delayed by more than 3 h with a mean of 4.50 h, which was found to be statistically significant. This shows that the inventive pharmaceutical composition was effective in delaying the peak plasma concentration of metoclopramide after oral administration.

The Cₘₐₓ level (maximum plasma concentration after administration) of the immediate release tablets has a value of 89.0 ng/ml, which is very high, relative to the Cₘₐₓ value for the modified release tablet of the invention (52.86 ng/ml). This shows that the modified release matrix provided a significant reduction in the maximum plasma concentrations.
This finding assures that the goal of modified release preparation has been attained, in reducing the high peak plasma concentrations and thereby, reducing the side effects, especially the extrapyramidal symptoms.

The ratio Cₘₐₓ/AUC_{0→∞} (AUC_{0→t} is the area under the plasma concentration versus time curve, from the time of administration and till the last observed plasma concentration at time t. AUC_{0→∞} is the area under the plasma concentration versus time curve extrapolated to infinite time, calculated as AUC_{0→t} + Cₜ/λz, where Cₜ is the last measurable drug concentration and λz is the terminal or elimination rate constant) is held to be a good parameter for evaluation of the absorption rate of prolonged release formulations. A statistically significant difference was found in this ratio between the two preparations, where the modified release matrix of the invention showed a slower rate of absorption. As expected, this shows that the release of metoclopramide from the pharmaceutical composition of the invention is slower than that of the conventional formulation.

The modified release characteristics of the matrix were reflected in the MRT (MRT is the mean residence time, calculated as AUMC/AUC). MRT is a useful parameter, especially in cases were the drug (such as metoclopramide) is rapidly eliminated. The MRT was noticeably increased following oral administration of the modified release tablet according to the invention (11.78 h) as compared to the immediate release tablets of the prior art (5.54 h). (AUMC is the area under the first moment curve, it is obtained from the plot of the product of the drug concentration in plasma and time versus time [C(t) vs t]).

As can be seen from the presented data, 55.3% of the maximum concentration remains in the body 8 h after administration of the inventive pharmaceutical composition and 30.1% remains after 12 h as compared to 24.4 and 9.3%, respectively, for the immediate release tablets. At both times, the percentage remained in the body after administration of the modified release tablets of the invention was found to be statistically significant when compared to that remaining after immediate release tablets administration.

The therapeutic range for plasma metoclopramide concentrations and the relationship of plasma concentration to clinical response and toxicity have not been clearly established. However, metoclopramide induced akathisia is reportedly associated with peak plasma metoclopramide concentrations greater than 120 ng/ml. The maximum plasma concentration of metoclopramide obtained in the controlled release study is clearly lower than the value indicating the safety of the reported formulation.

Figure 2 shows the metoclopramide plasma concentration time profiles after administration of the modified release tablets "example 1" according to the invention and the immediate release commercial tablets. The modified release tablets of "example 1" show a Cₘₐₓ of about 50 ng/ml, meanwhile the immediate release commercial tablets show a Cₘₐₓ of about 90 ng/ml. This difference was found statistically significant at the 95% confidence level. This shows that the inventive pharmaceutical composition provides a significant reduction in the maximum plasma concentrations, and thus a much less possibility for plasma levels causing extrapyrimidal symptoms is reached.

The features disclosed in the foregoing description, in the claims and/or the accompanying drawings may, both separately and in any combination thereof, be material for realizing the invention in diverse forms thereof.

## Claims

1. Pharmaceutical composition comprising at least one hydrophilic polymer as a matrix and metoclopramide dispersed therein.

2. Pharmaceutical composition according to claim 1, wherein the polymer is a natural polysaccharide.

3. Pharmaceutical composition according to claim 2, wherein the polymer is chitosan, sodium alginate or a mixture thereof.

4. Pharmaceutical composition according to any of the preceding claims, wherein metoclopramide is present in an amount of about 1 to about 50 weight percent, preferably about 5 to about 20 weight percent, based on the total weight of the pharmaceutical composition.

5. Pharmaceutical composition according to any of the preceding claims, wherein the polymer is present in an amount of about 10 to about 75 weight percent, preferably about 25 to about 75 weight percent, more preferably about 40 to about 60 weight percent, based on the total weight of the pharmaceutical composition.

6. Pharmaceutical composition according to any of the preceding claims, wherein at least one processing aid is added.

7. Pharmaceutical composition according to claim 6, wherein the processing aid comprises filler, binder, glidant, lubricant and/or disintegrant.

8. Pharmaceutical composition according to claim 7, wherein the pharmaceutical composition comprises about 5 to about 30 weight percent, preferably about 15 to about 30 weight percent filler, about 1 to about 15 weight percent, preferably about 5 to about 15 weight percent binder, about 0.5 to about 3 weight percent, preferably 0.5 to about 2 weight percent glidant, about 0.5 to about 3 weight percent, preferably 0.5 to about 2 weight percent lubricant and about 5 to about 20 weight percent disintegrant.

9. Pharmaceutical composition according to any of the preceding claims, wherein the composition is a capsule or a tablet.

10. Pharmaceutical composition according to claim 9, wherein the tablet is film coated.

11. Process for preparing a pharmaceutical composition according to any of the preceding claims 1 to 10, comprising the steps of mixing the polymer, metoclopramid and optionally processing aid(s), wet granulating the obtained mixture and compressing the obtained mixed granules into tablets.
